# EUROPÄISCHE PATENTSCHRIFT

(11) **EP 0 470 616 B1**
(45) Veröffentlichungstag und Bekanntmachung des Hinweises auf die Patenterteilung: **19.02.1997**
(21) Anmeldenummer: 91113334.6
(22) Anmeldetag: 08.08.1991
(51) Int. Cl.: C07D 239/42

(54) **Substituierte Pyrimidin-Derivate, Verfahren zu ihrer Herstellung und ihre Verwendung als Reagenzien**
Substituted pyrimidin derivatives, process for their preparation and their use as reagent
Dérivés de pyrimidine substitués, procédé pour leur préparation et leur utilisation comme réactifs

(30) Priorität: 10.08.1990 DE 4025387
(43) Veröffentlichungstag der Anmeldung: 12.02.1992
(73) Patentinhaber: HOECHST AKTIENGESELLSCHAFT, 65926 Frankfurt am Main (DE)
(72) Erfinder: Geisen, Karl, Dr., W-6000 Frankfurt a.M. (DE); Utz, Roland, Dr., W-6101 Messel (DE); Nimmesgern Hildegard, Dr., W-6000 Frankfurt a.M. (DE); Lang, Hans-Jochen, Dr., W-6238 Hofheim/Ts. (DE)

(56) Entgegenhaltungen:
- EP-A- 0 198 583
- EP-A- 0 384 370
- Arzneimittel Forsch./Drug Research 44(II), 1032-1042, (1994)

## Beschreibung

Bei diabetischen Individuen (Tier, Mensch) kommt es zum Auftreten erhöhter intrazellulärer Sorbit-Konzentrationen. Sorbit wird durch das Enzym Aldose-Reduktase bei erhöhter Blutglukose vermehrt gebildet. Die Sorbitakkumulation kann durch Aldose-Reduktasehemmer verhindert werden.

Das Screening nach Aldose-Reduktase Inhibitoren (ARI) erfolgt an Streptozotocin-diabetischen Ratten. 1 bis 2 Wochen nach Diabetesinduktion mit 60 mg Streptozotocin-Sulfat pro kg/Ratte werden die Tiere im ARI-Screening eingesetzt. Als Maß der Wirksamkeit von Aldose-Reduktase-Inhibitoren dient die Absenkung des erhöhten Sorbitgehalts in Erythrozyten, in Nerven und der Linse 5 - 6 h nach Behandlung mit den zu untersuchenden ARIs.

Streptozotocin ist ein Cancerogen. Applikation von Streptozotocin und Haltung der Tiere nach Applikation (2-3 Tage) müssen daher unter Biohazard-Bedingungen erfolgen. Der während der ersten 2 Tage nach Streptozotocin-Applikation ausgeschiedene Urin muß besonders entsorgt werden, die kontaminierten Boxen müssen speziell gereinigt werden. Streptozotocin wirkt aber nicht nur cancerogen bzw. toxisch auf β-Zellen des Pancreas, es verursacht auch Leber- und Nierenschäden. Deshalb werden die Tiere erst 10 - 14 Tage nach Applikation im ARI-Screening eingesetzt.

Die Verwendung von Pyrimidin-Derivaten der Formel I' 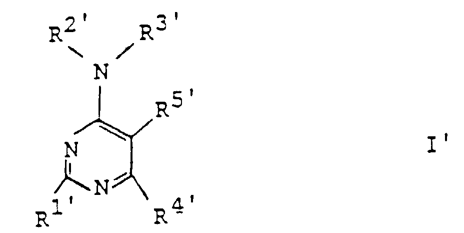 in welcher
- R^{1'}, R^{4'} und R^{5'}: gleich oder verschieden sind und Wasserstoff, Halogen, Cyano, Nitro, Trifluormethyl, (C₁-C₆)-Alkyl, (C₁-C₆)-Hydroxyalkyl, (C₁-C₆)-Alkoxy, (C₆-C₁₂)-Ary) oder Amino bedeuten,
- R^{2'}, R^{3'}: gleich oder verschieden sind und Wasserstoff, (C₁-C₆)-Alkyl, (C₆-C₁₂)-Ary) oder (C₆-C₁₂)-Aralkyl mit 1-4 Alkylkohlenstoffatomen sind, oder R^{2'} und R^{3'} bilden zusammen mit dem Stickstoff, an den sie gebunden sind, die Azetidino-, Pyrrolidino-, Piperidino-, Piperazino- oder Morpholinogruppe, oder eine mit gleichen oder verschiedenen Gruppen R^{6'} und R^{7'} substituierte Azetidino-, Pyrrolidino-, Piperidino-, Piperazino- oder Morpholinogruppe, wobei R^{6'}, R^{7'} (C₁-C₆)-Alkyl, Sulfamoyl, N-(C₁-C₄)-Alkylsulfamoyl, N,N-(C₁-C₄)-Dialkylsulfamoyl, (C₁-C₆)-Alkoxycarbonyl, N,N-(C₁-C₄)-Dialkylcarbamoyl, N-(C₁-C₄)-Alkylcarbamoyl, N-(C₆-C₁₂)-Arylcarbamoyl, im Arylrest mit (C₁-C₄)-Alkyl, (C₁-C₄)-Alkoxy, Halogen, NO₂, NH₂, CN oder CF₃ substituiertes (C₆-C₁₂)-Arylcarbamoyl, Carbamoyl, (C₁-C₆)-Alkylcarbonyl, (C₆-C₁₂)-Arylcarbonyl, im Arylrest mit (C₁-C₄)-Alkyl, (C₁-C₄)-Alkoxy, Halogen NO₂, NH₂, CN oder CF₃ substituiertes (C₆-C₁₂)-Arylcarbamoyl, (C₁-C₆)-Alkylcarbonyl, (C₁-C₆)-Alkylsulfinyl, (C₆-C₁₂)-Arylcarbonyl, im Arylrest mit (C₁-C₄)-Alkyl, (C₁-C₄)-Alkoxy, Halogen, NO₂, NH₂, CN oder CF₃ substituiertes (C₆-C₁₂)-Arylsulfonyl, Heteroarylcarbonyl oder Heteroarylsulfonyl bedeuten oder einer der Substituenten R^{6'}, R^{7'} Wasserstoff ist, sowie von deren physiologisch verträglichen Salzen als Tool wurde bereits vorgeschlagen (vergl. Deutsche Offenlegungschrift 39 05 364 entsprechend EP-A-O 384 370 bzw. U.S. patentanmeldung Nr. 07/482053). Entsprechend den Angaben in dieser Patentanmeldung verursachen dort beschriebene Verbindungen ohne Einfluß auf die Blutglukose akut und chronisch, oral oder parenteral an normale, nicht diabetische Ratten verabreicht einen intrazellulären Sorbitanstieg, der durch simultane Behandlung mit Aldose-Reductase-Inhibitoren verhindert werden kann.

Es wurde nun überraschenderweise gefunden, daß die in der deutschen Offenlegungsschrift 39 05 364 nicht expressis verbis genannten Pyrimidin-Derivate der Formel I 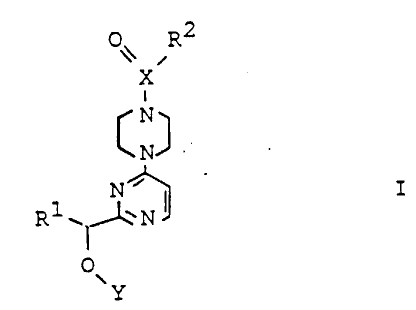 und deren pharmakologisch verträgliche Salze wesentlich günstigere Eigenschaften besitzen. Sie verursachen ohne Einfluß auf die Blutglukose akut und chronisch, oral oder parenteral verabreicht einen intrazellulären Sorbitanstieg. Der durch die Pyrimidin-Derivate der Formel I induzierte Sorbitanstieg wird durch simultane Behandlung mit Aldose-Reduktase-Inhibitoren verhindert. Die Sorbit-akkumulierenden Pyrimidin-Derivate der Formel I eignen sich daher für ein neues, vereinfachtes, weniger kosten- und zeitaufwendiges Akut-Screening nach Aldose-Reductase-Inhibitoren an normalen, nicht diabetischen Ratten. Im Gegensatz zu den in der deutschen Patentanmeldung genannten Beispielen zeigen die Verbindungen der obigen Formel I in vivo außer an der Ratte auch an anderen Spezies eine Sorbit-akkumulierende Wirkung. Ein ganz erheblicher, nicht zu erwartender Vorteil besteht darin, daß Verbindungen der Formel I nicht nur in vivo sondern auch in vitro, Z.B. an Erythrocyten, Sorbitolerhöhungen induzieren und somit ein noch kostengünstigeres und leichter durchführbares Testmodell für z.B Aldose-Reduktasehemmer realisierbar ist.

Die vorliegende Erfindung betrifft daher Pyrimidin-Derivate der Formel I 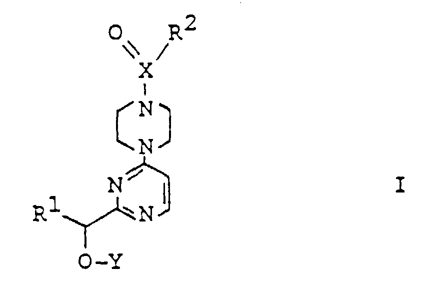 worin
- R¹: Wasserstoff oder Methyl ist
- R²: C₁-C₆-Alkyl, C₅-C₇-Cycloalkyl, unsubstituiertes Phenyl, mit Methyl, Chlor, Fluor, Trifluormethyl, Methoxy, CH₃-SOₙ mit n gleich null, 1 oder 2, oder -SO₂-NH₂ mono- oder disubstituiertes Phenyl, wobei im Falle der Disubstitution die Substituenten gleich oder verschieden sind, 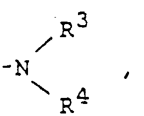 wobei R³ und R⁴ gleich oder verschieden sind und Wasserstoff oder C₁-C₆-Alkyl bedeuten, oder Pyridyl bedeutet oder, falls X ein C-Atom ist, auch Wasserstoff darstellt,
- X: ein Kohlenstoffatom oder SO ist und
- Y: Wasserstoff ist,
sowie deren physiologisch verträgliche Salze.

Die unter Y und R² der Formel I bezeichneten Alkylreste können geradkettig oder verzweigt sein.

Bevorzugt sind Pyrimidin-Derivate der Formel I, worin
a) R¹ Wasserstoff ist,
   - R²: C₁-C₃-Alkyl, unsubstituiertes Phenyl oder mono- oder disubstituiertes Phenyl, wobei die Substituenten die angegebenen Bedeutungen haben, darstellt und X und Y die angegebenen Bedeutungen haben,
b) R¹ Wasserstoff ist 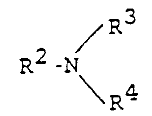 wobei R³ und R⁴ gleich oder verschieden sind und Methyl oder Ethyl bedeuten, darstellt,
   - X: SO bedeutet und
   - Y: die angegebene Bedeutung hat,
c) Wasserstoff ist,
   - R¹:
   - R²: Pyridyl darstellt,
   - X: ein Kohlenstoffatom bedeutet und
   - Y: die angegebenen Bedeutung hat
   sowie deren physiologisch verträgliche Salze.

Ganz besonders bevorzugt ist das Pyrimidin-Derivat der Formel I worin R¹ Wasserstoff, 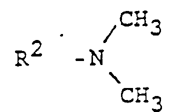 X SO und Y Wasserstoff bedeuten, sowie die physiologisch verträglichen Salze dieser Verbindung.

Die Erfindung betrifft weiterhin ein Verfahren zur Herstellung von Verbindungen der Formel I, das dadurch gekennzeichnet ist, daß man in an sich bekannter Weise
a) eine Verbindung der Formel II 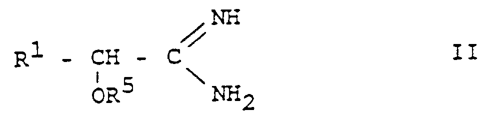
   worin R¹ die zur Formel I angegebene Bedeutung hat und R⁵ eine geeignete Ätherschutzgruppe, wie Methyl oder Benzyl, bedeutet, oder deren Säureadditionssalz, mit einer Verbindung der Formel III 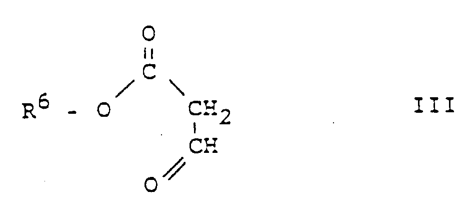 worin R⁶ Methyl oder Ethyl bedeutet, oder einem ihrer Alkalisalze, zu einer Verbindung der Formel IV 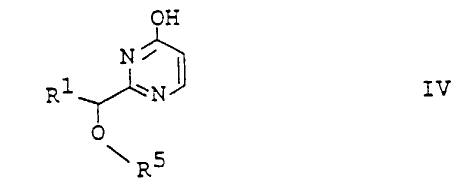 worin R¹ die zu Formel I und R⁵ die zu Formel II angegebenen Bedeutungen haben, umsetzt, eine erhaltene Verbindung der Formel IV mit einem anorganischen Säurechlorid, wie z.B. mit Phosphoroxychlorid, in ein Pyrimidin-Derivat der Formel V 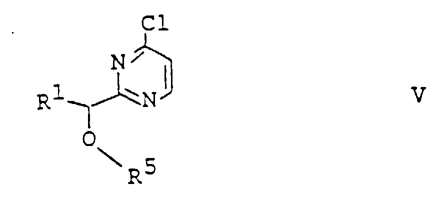 in welcher die Reste R¹ die zu Formel I und R⁵ die zu Formel II angegebene Bedeutung haben, überführt,
   und anschließend
   α) eine erhaltene Verbindung der Formel V mit einem Piperazin-Derivat der Formel VI 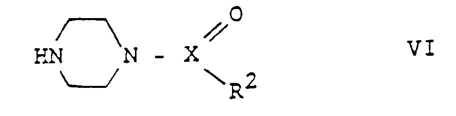 worin R² und X die zu Formel I angegebenen Bedeutungen besitzen, zu einer Verbindung der Formel VII 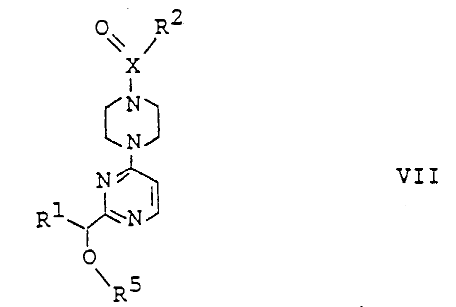 worin R¹, R² und X die zu Formel I und R⁵ die zu Formel II angegebene Bedeutung besitzen, umsetzt oder
   β) eine erhaltene Verbindung der Formel V mit unsubstituiertem Piperazin in eine Verbindung der Formel VIII 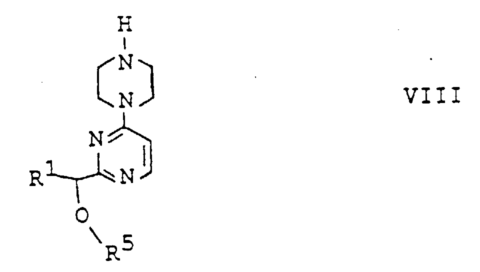 worin R¹ die zu Formel I, und R⁵ die zu Formel II angegebene Bedeutung besitzen, überführt und die erhaltene Verbindung VIII mit einem Säurechlorid der Formel IX 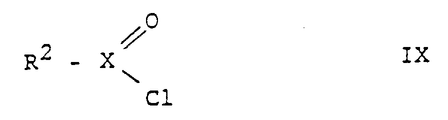 worin R² und X die zu Formel I angegebene Bedeutung besitzen, in eine Verbindung der Formel VII mit den angegebenen Bedeutungen der Substituenten überführt, und
   eine nach α) oder β) erhaltene Verbindung der Formel VII gegebenenfalls in an sich bekannter Weise durch ätherspaltendes Reagenz, beispielsweise mit BBr₃, Trimethylsilyljodid, oder durch katalytische Hydrierung, wenn R⁵ in der Bedeutung von Benzyl steht, in eine
   Verbindung der Formel I überführt, b) eine Verbindung der Formel X 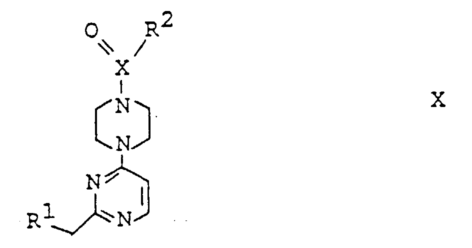 worin R¹, R² und X die zu Formel I angegebenen Bedeutungen haben,
   mit einem Halogenierungsmittel, wie Chlor, Brom, N-Bromsuccinimid, Sulfurylchlorid oder mit Trichlorisocyanursäure, in an sich bekannter Weise in eine Verbindung der Formel XI 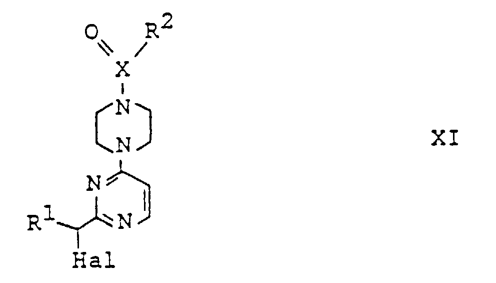 worin die Substituenten R¹, R² und X die zu Formel I angegebenen Bedeutungen haben und Hal für Cl oder Br steht, überführt,
   und eine erhaltene Verbindung der Formel XI entweder durch direkte Hydrolyse oder durch Reaktion mit einem Alkalisalz der Essig- oder Benzoesäure z.B. Natriumacetat oder Natriumbenzoat, oder dem Alkalisalz des Benzylalkohols oder dem Alkalisalz eines C₁-C₆-Alkylalkohols in eine Verbindung der Formel I überführt mit R¹, R², X und Y in den angegebenen Bedeutungen und gegebenenfalls eine nach Verfahren a) oder b) erhaltenen Verbindung der Formel I mit Y ungleich Wasserstoff in an sich bekannter Weise durch saure oder alkalische Hydrolyse in eine Verbindung der Formel I mit Y gleich Wasserstoff überführt, und gegebenenfalls eine erhaltene Verbindung der Formel I in ein physiologisch verträgliches Salz überführt.

Verbindungen der Formel VIII können in an sich bekannter Weise auch durch saure oder alkalische Hydrolyse der Verbindungen VII erhalten werden.

Das erfindungsgemäße Verfahren a) wird analog den in der Literatur beschriebenen Verfahren durchgeführt (vgl. z.B. D. J. Brown, The Chemistry of Heterocyclic Compunds, The Pyrimidines Suppl. I (1970), Suppl. II (1985), Wiley Interscience, N.Y. und darin zitierte Literatur).

Die Seitenkettenchlorierung des erfindungsgemäßen Verfahrens b) wird vorzugsweise mit Trichlorisocyanursäure analog den Angaben von G. E. Jeromin et al., Seitenkettenchlorierungen von N-Heterocyclen mit Trichlorcyanursäure (TCC) in Chem. Ber. 120, 649-651 (1987) durchgeführt.

Verbindungen der allgemeinen Formel I können durch Umsetzung mit Säuren in ihre physiologisch verträglichen Salze übergeführt werden.

Bislang mußten zur Untersuchung von Verbindungen mit Wirkung auf diabetische Spätschäden, beispielsweise von Aldose-Reduktasehemmern, diabetische Tiere verwendet werden. In diesen Tieren mußte eine künstliche diabetische Erkrankung durch Zerstörung der insulinsezernierenden β-Zellen mit toxischen Verbindungen, wie Streptozotocin, Alloxan usw., provoziert werden. Die irreversibel geschädigten Tiere, die - wie eingangs beschrieben-erhöhte Blutglucose- und intrazelluläre Sorbitspiegel entwickeln, sind infolge ihrer diabetischen Erkrankung nur mit aufwendigeren und kostenintensiveren Pflegemaßnamen zu halten und wegen ihrer Schwächung nur schwierig von sekundären Erkrankungen, die wiederum ihre Verwendbarkeit beeinträchtigen, freizuhalten.

Es war nun überraschend, daß die erfindungsgemäßen Verbindungen, die sich durch gute Verträglichkeit auszeichnen, in gesunden Tieren durch eine intrazelluläre Polyol-Akkumulation ohne vorausgehende diabetische Stoffwechsellage funktionelle Symptome im Sinne einer diabetischen Neurophathie erzeugen.

Aufgrund dieser Fähigkeit, eine Sorbit-Akkumulation zu bewirken, eignen sich die erfindungsgemäßen Verbindungen in vorteilhafter Weise als Reagenzien zur Prüfung von Aldose-Reduktasehemmern im pharmakologischen Modell an gesunden Tieren. Ferner eignen sich die Verbindungen der Formel I als in vitro-Reagenz für Aldose-Reduktasehemmer. Die Erfindung betrifft daher auch diese Verwendung der Pyrimidinderivate der Formel I und von deren pharmakologisch verträglichen Salzen.

In Dosen von 5 - 50 mg/kg Ratte oral verabreicht, verursachten die Verbindungen gemäß der vorliegenden Erfindung einen von der Dosis abhängigen Anstieg der Sorbitkonzentration im Ischiadicus-Nerv und in den Erythrocyten normaler und Streptozotocin-diabetischer Ratten innerhalb von 4 bis 5 Stunden.

Nach oraler Verabreichung von 25 mg/kg Ratte der Verbindung gemäß Beispiel 1 oder 2 wird nach 4 - 5 Stunden bei normalen Ratten eine Sorbitkonzentration in den genannten Geweben erreicht, die derjenigen entspricht, welche Streptozotocin-diabetische Ratten nach 8 Tagen aufweisen. Durch simultane orale Behandlung mit dem ARI Spiro-2,7-difluor-9H-fluoren-9,4-imidazolidin-2,5-dion (= HOE 843) wird der Sorbitanstieg dosisabhängig verhindert.

Außer den in den Beispielen aufgeführten Verbindungen können die in der folgenden Tabelle zusammengestellten Verbindungen der allgemeinen Formel I bzw. deren Salze hergestellt werden.

Verwendete Abkürzungen: Methyl (Me), Ethyl (Et), Propyl (Prop), Butyl (Bu),Hexyl (Hex), Acetyl (Ac), Phenyl (Ph), Benzoyl (Bz) iso (i) und cyclo (c).

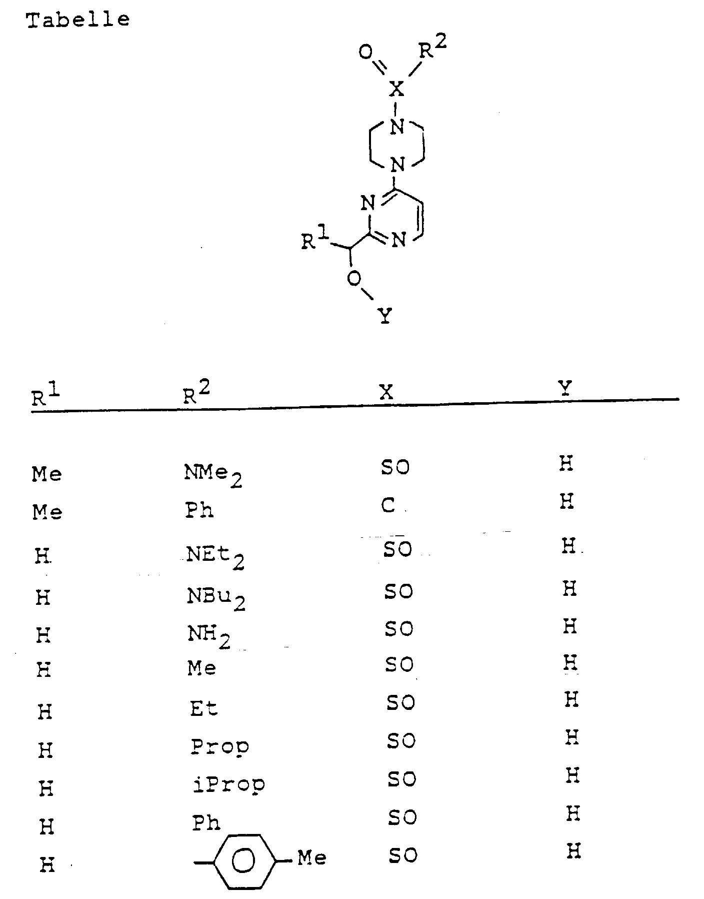

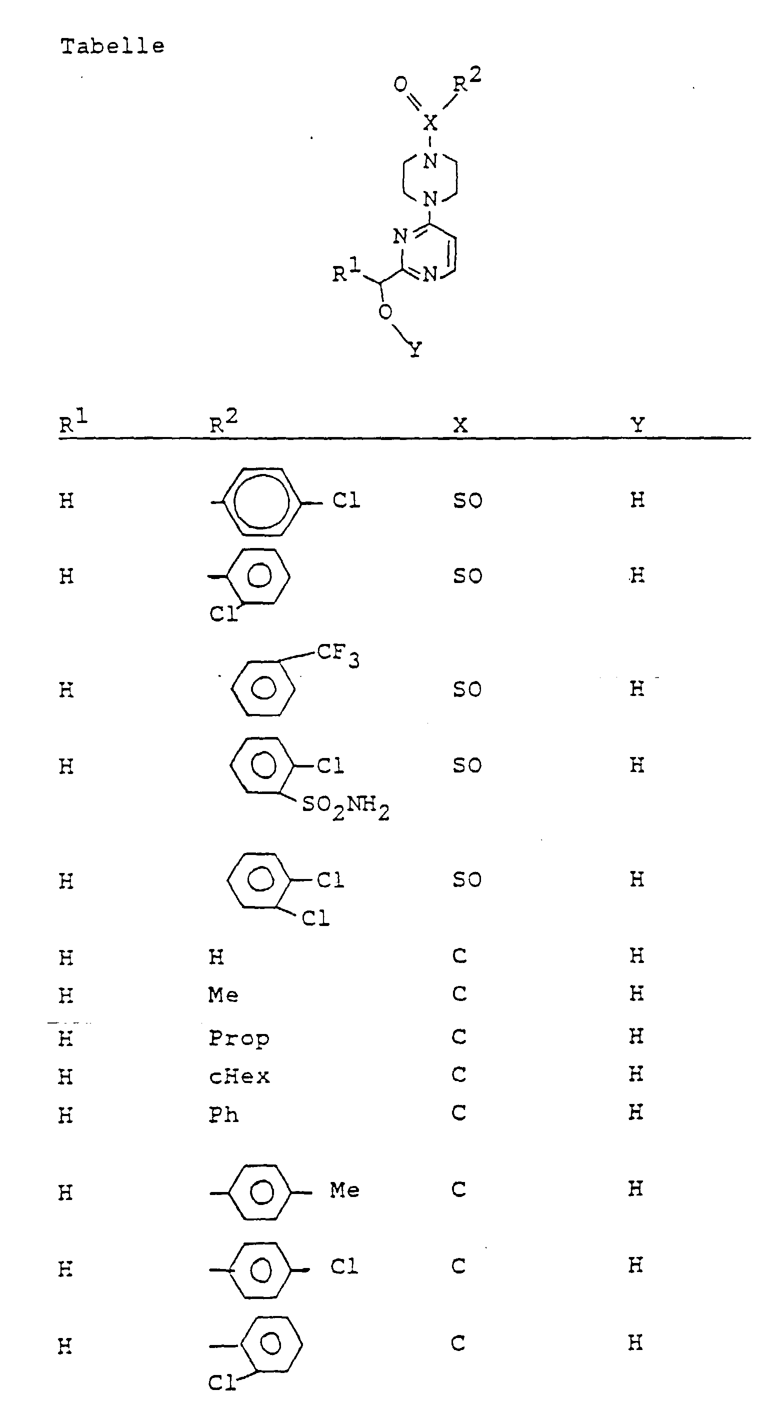

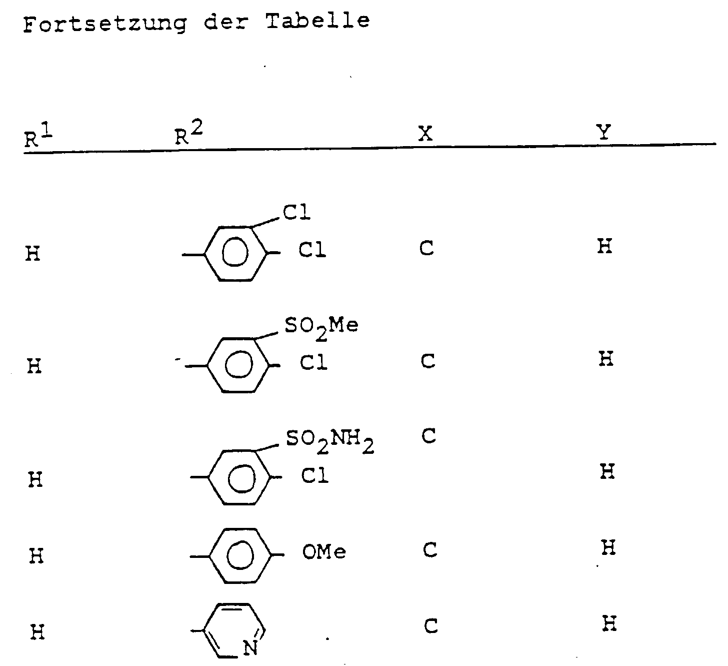

### Beispiel 1

4-[4-(N,N-Dimethylsulfamoyl)-piperazino]-2-hydroxymethylpyrimidin 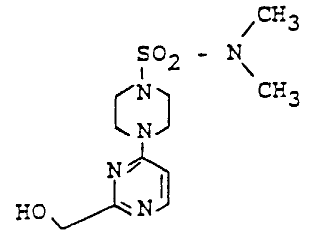 11,0 g (35 mmol) 4-[4-(N,N-Dimethylsulfamoyl)-piperazino]-2-nethyloxymethylpyrimidin (Beispiel 1) werden in 45 ml trockenem Methylenchlorid gelöst. Zwischen 0 ° bis + 5 °C werden 13,2 g (52,5 mmol) Bortribromid gelöst in 25 ml Methylenchlorid unter Argon langsam zugetropft. Man rührt 1,5 h unter Eiskühlung nach und hydrolysiert mit kalter 2 N Natronlauge. Man trennt die organische Phase ab und extrahiert die wässrige Phase noch 3 mal mit Methylenchlorid. Die vereinigten organischen Phasen werden mit gesättigter Ammoniumchloridlösung, Wasser und gesättigter Natriumchloridlösung gewaschen, über Natriumsulfat getrocknet und das Lösungsmittel wird i. Vak. entfernt. Nach Kristallisation des Rückstandes in Butylacetat erhält man 7,60 g festes Produkt vom Smp. 148-149 °C.

| | | | | | | | |
|---|---|---|---|---|---|---|---|
| C₁₁H₁₉N₅O₃S (301,38): | Ber.: | C | 43,84 | H | 6,35 | N | 23,24 |
| | Gef.: | C | 43,7 | H | 6,4 | N | 21,1 |

¹H-NNR (60 MHz/DMSO): δ = 8,23 (d, J =6Hz, 1H); δ = 6,75 (d, J=6Hz, 1H); δ = 4,82 (t, J=5Hz, 1H); δ = 3,73 (m, 4H); δ = 3,27 (m, 4H); δ = 2,78 (s, 6H).

## Patentansprüche (Patentansprüche für folgende(n) Vertragsstaat(en): AT, BE, CH, DE, DK, FR, GB, GR, IT, LI, LU, NL, SE)

1. Pyrimidin-Derivate der Formel I 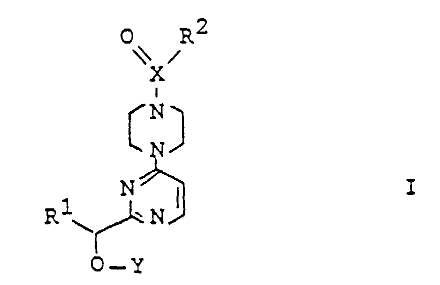 worin
R¹ Wasserstoff oder Methyl ist
R² C₁-C₆-Alkyl, C₅-C₇-Cycloalkyl, unsubstituiertes Phenyl, mit Methyl, Chlor, Fluor, Trifluormethyl, Methoxy, CH₃-SOₙ mit n gleich null, 1 oder 2, oder -SO₂-NH₂ mono- oder disubstituiertes Phenyl, wobei im Falle der Disubstitution die Substituenten gleich oder verschieden sind, 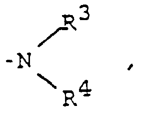 wobei R³ und R⁴ gleich oder verschieden sind und Wasserstoff oder C₁-C₆-Alkyl bedeuten, oder Pyridyl bedeutet oder, falls X ein C-Atom ist, auch Wasserstoff darstellt,
X ein Kohlenstoffatom oder SO ist und
Y Wasserstoff ist,
sowie deren physiologisch verträgliche Salze.

2. Pyrimidin-Derivate gemäß Anspruch 1, dadurch gekennzeichnet, daß in Formel I
R¹ Wasserstoff ist,
R² C₁-C₃-Alkyl, unsubstituiertes Phenyl oder mono- oder disubstituiertes Phenyl, wobei die Substituenten die angegebenen Bedeutungen haben, darstellt und
X und Y die angegebenen Bedeutungen haben.

3. Pyrimidin-Derivate gemäß Anspruch 1, dadurch gekennzeichnet, daß in Formel I
R¹ Wasserstoff ist 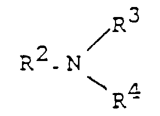 wobei R³ und R⁴ gleich oder verschieden sind und Methyl oder Ethyl bedeuten, darstellt,
X SO bedeutet und
Y die angegebene Bedeutung hat.

4. Pyrimidin-Derivate gemäß Anspruch 1, dadurch gekennzeichnet, daß in Formel I
R¹ Wasserstoff ist,
R² Pyridyl darstellt,
X ein Kohlenstoffatom bedeutet und
Y die angegebenen Bedeutung hat

5. Pyrimidin-Derivat der in Anspruch 1 angegebenen Formel I worin R¹ Wasserstoff, 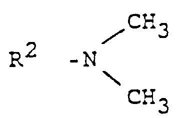 X SO und Y Wasserstoff bedeuten, sowie die physiologisch verträglichen Salze dieser Verbindung.

6. Verfahren zur Herstellung von Verbindungen gemäß Anspruch 1, dadurch gekennzeichnet, daß man in an sich bekannter Weise
a) eine Verbindung der Formel II 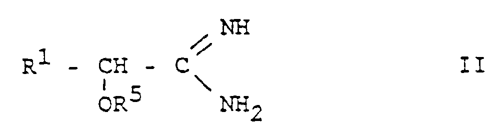 worin R¹ die zur Formel I angegebene Bedeutung hat und R⁵ eine geeignete Ätherschutzgruppe bedeutet, oder deren Säureadditionssalz, mit einer Verbindung der Formel III 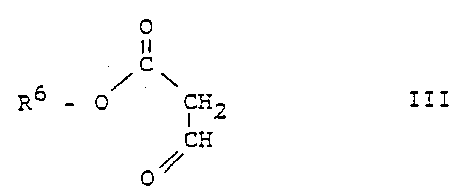 worin R⁶ Methyl oder Ethyl bedeutet, oder einem ihrer Alkalisalze, zu einer Verbindung der Formel IV 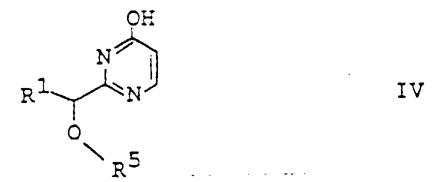 worin R¹ die zu Formel I und R⁵ die zu Formel II angegebenen Bedeutungen haben, umsetzt, eine erhaltene Verbindung der Formel IV mit einem anorganischen Säurechlorid in ein Pyrimidin-Derivat der Formel V 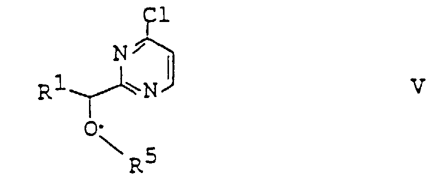 in welcher die Reste R¹ die zu Formel I und R⁵ die zu Formel II angegebene Bedeutung haben, überführt,
und anschließend
α) eine erhaltene Verbindung der Formel V mit einem Piperazin-Derivat der Formel VI 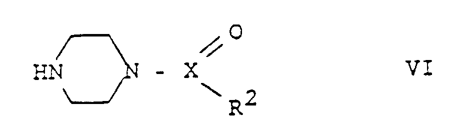 worin R² und X die zu Formel I angegebenen Bedeutungen besitzen, zu einer Verbindung der Formel VII 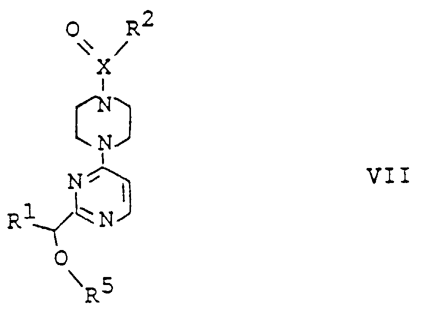 worin R¹, R² und X die zu Formel I und R⁵ die zu Formel II angegebene Bedeutung besitzen, umsetzt oder
β) eine erhaltene Verbindung der Formel V mit unsubstituiertem Piperazin in eine Verbindung der Formel VIII 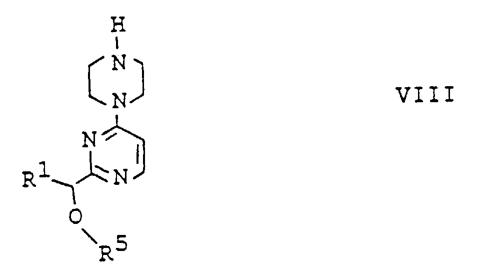 worin R¹ die zu Formel I, und R⁵ die zu Formel II angegebene Bedeutung besitzen, überführt und die erhaltene Verbindung VIII mit einem Säurechlorid der Formel IX 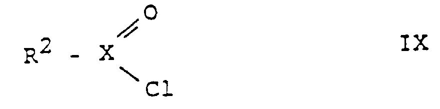 worin R² und X die zu Formel I angegebene Bedeutung besitzen, in eine Verbindung der Formel VII mit den angegebenen Bedeutungen der Substituenten überführt, und eine nach α) oder β) erhaltene Verbindung der Formel VII gegebenenfalls in an sich bekannter Weise durch ätherspaltendes Reagenz, oder durch katalytische Hydrierung, wenn R⁵ in der Bedeutung von Benzyl steht, in eine Verbindung der Formel I überführt,
b) eine Verbindung der Formel X 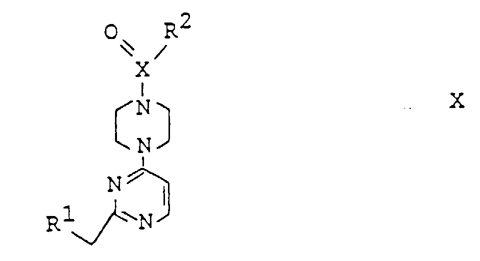 worin R¹, R² und X die zu Formel I angegebenen Bedeutungen haben,
mit einem Halogenierungsmittel in an sich bekannter Weise in eine Verbindung der Formel XI 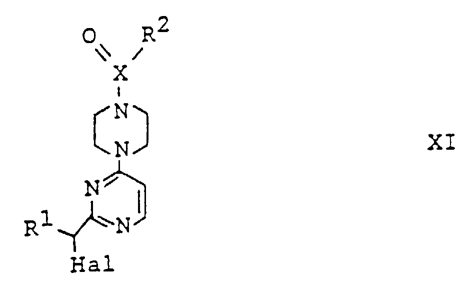 worin die Substituenten R¹, R² und X die zu Formel I angegebenen Bedeutungen haben und Hal für Cl oder Br steht, überführt,
und eine erhaltene Verbindung der Formel XI entweder durch direkte Hydrolyse oder durch Reaktion mit einem Alkalisalz der Essig- oder Benzoesäure oder dem Alkalisalz des Benzylalkohols oder dem Alkalisalz eines C₁-C₆-Alkylalkohols in eine Verbindung der Formel I überführt mit R¹, R², X und Y in den angegebenen Bedeutungen und gegebenenfalls eine nach Verfahren a) oder b) erhaltenen Verbindung der Formel I mit Y ungleich Wasserstoff in an sich bekannter Weise durch saure oder alkalische Hydrolyse in eine Verbindung der Formel I mit Y gleich Wasserstoff überführt und gegebenenfalls eine erhaltene Verbindung der Formel I in ein physiologisch verträgliches Salz überführt.

7. Verwendung von Verbindungen gemäß Anspruch 1 als Reagenz.

## Patentansprüche (Patentansprüche für folgende(n) Vertragsstaat(en): ES)

1. Verfahren zur Herstellung von Pyrimidin-Derivaten der Formel I 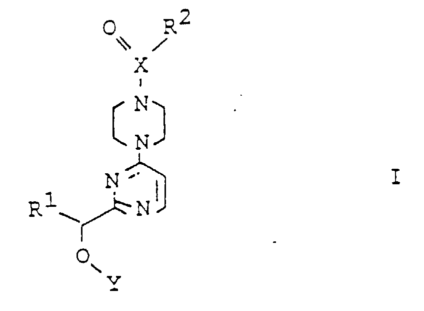 worin
R¹ Wasserstoff oder Methyl ist
R² C₁-C₆-Alkyl, C₅-C₇-Cycloalkyl, unsubstituiertes Phenyl, mit Methyl, Chlor, Fluor, Trifluormethyl, Methoxy, CH₃-SOₙ mit n gleich null, 1 oder 2, oder -SO₂-NH₂ mono- oder disubstituiertes Phenyl, wobei im Falle der Disubstitution die Substituenten gleich oder verschieden sind, 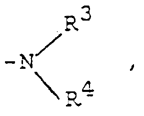 wobei R³ und R⁴ gleich oder verschieden sind und Wasserstoff oder C₁-C₆-Alkyl bedeuten, oder Pyridyl bedeutet oder, falls X ein C-Atom ist, auch Wasserstoff darstellt,
X ein Kohlenstoffatom oder SO ist und
Y Wasserstoff,
ist,
sowie von deren physiologisch verträglichen Salzen, dadurch gekennzeichnet, daß man
a) eine Verbindung der Formel II 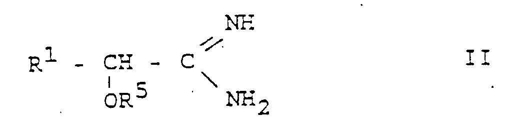 worin R¹ die zur Formel I angegebene Bedeutung hat und R⁵ eine geeignete Ätherschutzgruppe bedeutet, oder deren Säureadditionssalz, mit einer Verbindung der Formel III 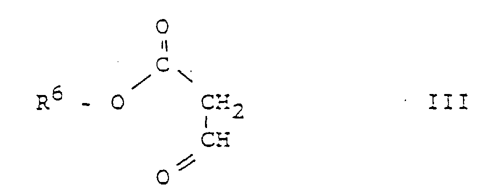 worin R⁶ Methyl oder Ethyl bedeutet, oder einem ihrer Alkalisalze, zu einer Verbindung der Formel IV 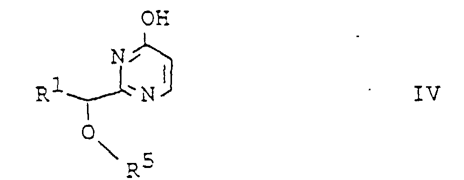 worin R¹ die zu Formel I und R⁵ die zu Formel II angegebenen Bedeutungen haben, umsetzt,
eine erhaltene Verbindung der Formel IV mit einem anorganischen Säurechlorid in ein Pyrimidin-Derivat der Formel V 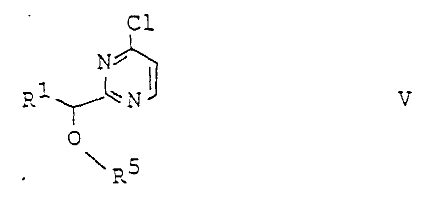 in welcher die Reste R¹ die zu Formel I und R⁵ die zu Formel II angegebene Bedeutung haben, überführt,
und anschließend
α) eine erhaltene Verbindung der Formel V mit einem Piperazin-Derivat der Formel VI 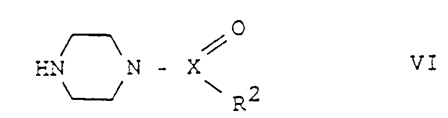 worin R² und X die zu Formel I angegebenen Bedeutungen besitzen, zu einer Verbindung der Formel VII 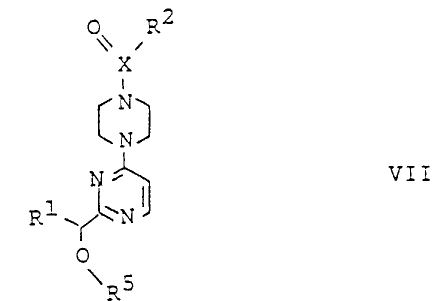 worin R¹, R² und X die zu Formel I und R⁵ die zu Formel II angegebene Bedeutung besitzen, umsetzt oder
β) eine erhaltene Verbindung der Formel V mit unsubstituiertem Piperazin in eine Verbindung der Formel VIII 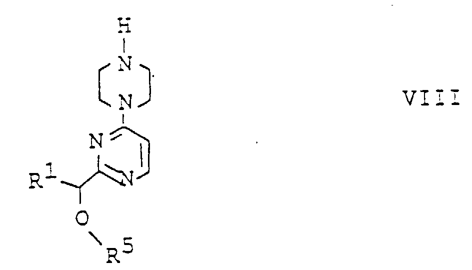 worin R¹ die zu Formel I, und R⁵ die zu Formel II angegebene Bedeutung besitzen, überführt und die erhaltene Verbindung VIII mit einem Säurechlorid der Formel IX 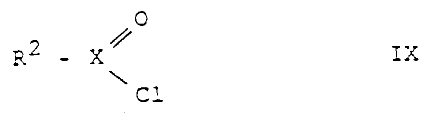 worin R² und X die zu Formel I angegebene Bedeutung besitzen, in eine Verbindung der Formel VII mit dem angegebenen Bedeutungen der Substituenten überführt, und eine nach α) oder β) erhaltene Verbindung der Formel VII gegebenenfalls in an sich bekannter Weise durch ätherspaltendes Reagenz, oder durch katalytische Hydrierung, wenn R⁵ in der Bedeutung von Benzyl steht, in eine Verbindung der Formel I überführt,
b) eine Verbindung der Formel X 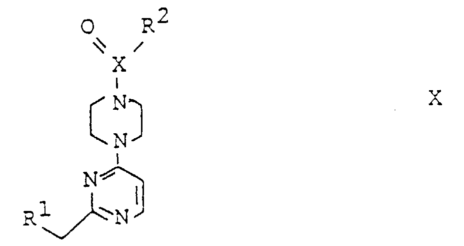 worin R¹, R² und X die zu Formel I angegebenen Bedeutungen haben,
mit einem Halogenierungsmittel in eine Verbindung der Formel XI 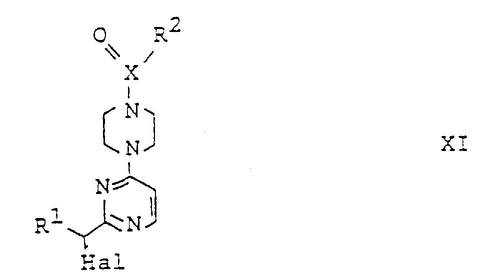 worin die Substituenten R¹, R² und X die zu Formel I angegebenen Bedeutungen haben und Hal für Cl oder Br steht, überführt,
und eine erhaltene Verbindung der Formel XI entweder durch direkte Hydrolyse oder durch Reaktion mit einem Alkalisalz der Essig- oder Benzoesäure oder dem Alkalisalz des Benzylalkohols oder dem Alkalisalz eines C₁-C₆ Alkylalkohols in eine Verbindung der Formel I überführt mit R¹, R², X und Y in den angegebenen Bedeutungen und gegebenenfalls eine nach Verfahren a) oder b) erhaltenen Verbindung der Formel I mit Y ungleich Wasserstoff in an sich bekannter Weise durch saure oder alkalische Hydrolyse in eine Verbindung der Formel I mit Y gleich Wasserstoff überführt und gegebenenfalls eine erhaltene Verbindung der Formel I in ein physiologisch verträgliches Salz überführt.

2. Verfahren gemäß Anspruch 1, dadurch gekennzeichnet, daß man eine Verbindung der Formel I, worin
R¹ Wasserstoff ist,
R² C₁-C₃-Alkyl, unsubstituiertes Phenyl oder mono- oder disubstituiertes Phenyl, wobei die Substituenten die angegebenen Bedeutungen haben, darstellt und
X und Y die angegebenen Bedeutungen haben, oder deren physiologisch verträgliche Salze herstellt.

3. Verfahren gemäß Anspruch 1, dadurch gekennzeichnet, daß man eine Verbindung der Formel I, worin
R¹ Wasserstoff ist 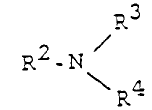 wobei R³ und R⁴ gleich oder verschieden sind und Methyl oder Ethyl bedeuten, darstellt,
X SO bedeutet und
Y die angegebene Bedeutung hat,
oder deren physiologisch verträgliche Salze herstellt.

4. Verfahren gemäß Anspruch 1, dadurch gekennzeichnet, daß man eine Verbindung der Formel I, worin
R1 Wasserstoff ist,
R² Pyridyl darstellt,
X ein Kohlenstoffatom bedeutet und
Y die angegebenen Bedeutung hat,
oder deren physiologisch verträgliche Salze herstellt.

5. Verfahren gemäß Anspruch 1, dadurch gekennzeichnet, daß man eine Verbindung der Formel
I worin R¹ Wasserstoff, 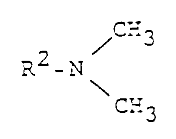 X SO und Y Wasserstoff bedeuten, oder die physiologisch verträglichen Salze dieser Verbindung herstellt.

6. Verwendung von Verbindungen erhältlich gemäß Anspruch 1 als Reagenz.

## Claims (Claims for the following Contracting State(s): AT, BE, CH, DE, DK, FR, GB, GR, IT, LI, LU, NL, SE)

1. A pyrimidine derivative of the formula I 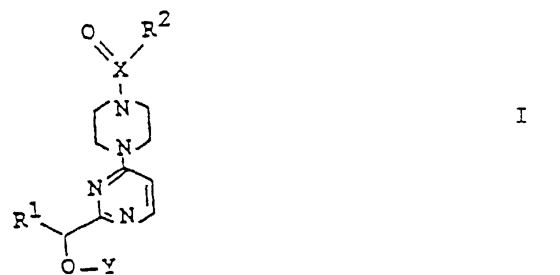 in which
R¹ is hydrogen or methyl,
R² is C₁-C₆-alkyl, C₅-C₇-cycloalkyl, unsubstituted phenyl, phenyl which is mono- or disubstituted by methyl, chlorine, fluorine, trifluoromethyl, methoxy, CH₃-SOₙ with n equal to zero, 1 or 2, or -SO₂-NH₂, where in the case of disubstitution the substituents are identical or different, 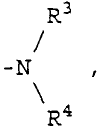 where R³ and R⁴ are identical or different and are hydrogen or C₁-C₆-alkyl, or is pyridyl or, if X is a carbon atom, is also hydrogen,
X is a carbon atom or SO, and
Y is hydrogen, and the physiologically tolerated salts thereof.

2. A pyrimidine derivative as claimed in claim 1, wherein in formula I
R¹ is hydrogen,
R² is C₁-C₃-alkyl, unsubstituted phenyl or mono- or disubstituted phenyl, where the substituents have the stated meanings, and
X and Y have the stated meanings.

3. A pyrimidine derivative as claimed in claim 1, wherein in formula I
R¹ is hydrogen,
R² is 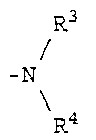 where R³ and R⁴ are identical or different and are methyl or ethyl,
X is SO, and
Y has the stated meaning.

4. A pyrimidine derivative as claimed in claim 1, wherein in formula I
R¹ is hydrogen,
R² is pyridyl,
X is a carbon atom, and
Y has the stated meaning.

5. A pyrimidine derivative of the formula I indicated in claim 1, in which R¹ is hydrogen, R² is 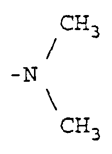 X is SO and Y is hydrogen, and the physiologically tolerated salts of this compound.

6. A process for preparing compounds as claimed in claim 1, which comprises in a manner known per se
a) reacting a compound of the formula II 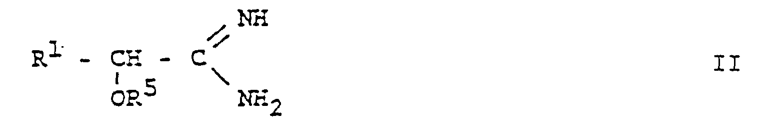 in which R¹ has the meaning stated for formula I, and R⁵ is a suitable ether protective group, or the acid addition salt thereof, with a compound of the formula III 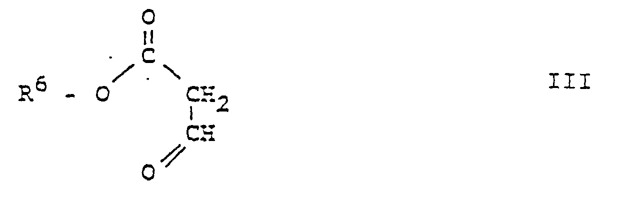 in which R⁶ is methyl or ethyl, or one of the alkali metal salts thereof, to give a compound of the formula IV 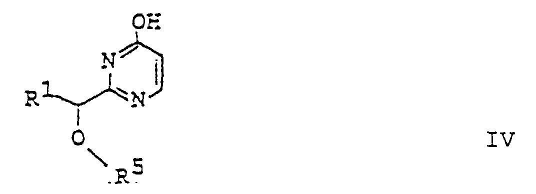 in which R¹ has the meanings stated for formula I and R⁵ has the meanings stated for formula II, converting a resulting compound of the formula IV with an inorganic acid chloride into a pyrimidine derivative of the formula V 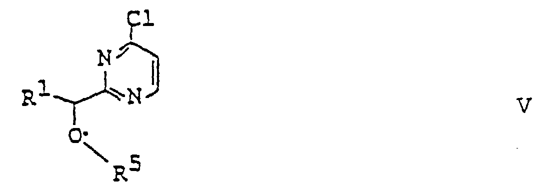 in which the radicals R¹ have the meaning stated for formula I and R⁵ has the meaning stated for formula II,
and subsequently,
α) reacting a resulting compound of the formula V with a piperazine derivative of the formula VI 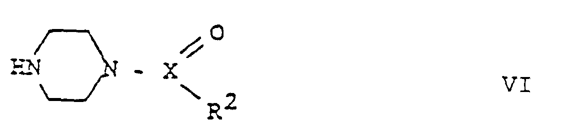 in which R² and X have the meanings stated for formula I, to give a compound of the formula VII 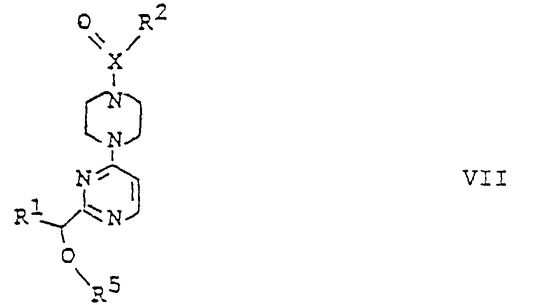 in which R¹, R² and X have the meaning stated for formula I and R⁵ has the meaning stated for formula II, or
β) converting a resulting compound of the formula V with unsubstituted piperazine into a compound of the formula VIII 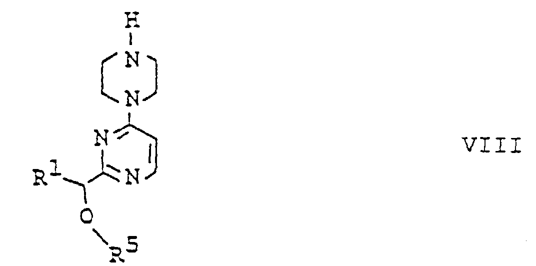 in which R¹ has the meaning stated for formula I and R⁵ has the meaning stated for formula II, and converting the resulting compound VIII with an acid chloride of the formula IX 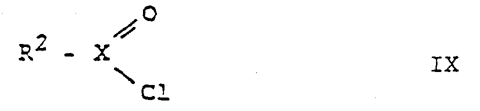 in which R² and X have the meaning stated for formula I, into a compound of the formula VII with the stated meanings of the substituents, and converting a compound of the formula VII obtained as in α) or β) where appropriate in a manner known per se by an ether-cleaving reagent, or by catalytic hydrogenation, when R⁵ has the meaning of benzyl, into a compound of the formula I,
b) converting a compound of the formula X 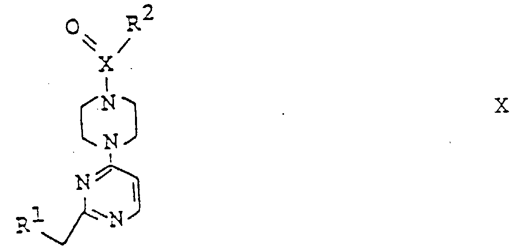 in which R¹, R² and X have the meanings stated for formula I,
with a halogenating agent in a manner known per se into a compound of the formula XI 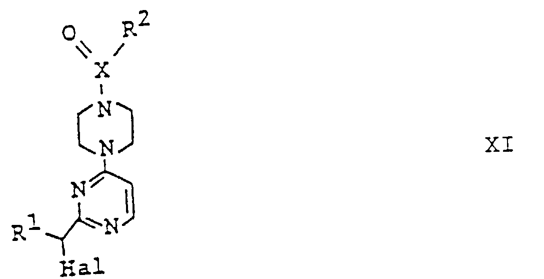 in which the substituents R¹, R² and X have the meanings stated for formula I, and Hal is Cl or Br, and converting a resulting compound of the formula XI either by direct hydrolysis or by reaction with an alkali metal salt of acetic or benzoic acid or the alkali metal salt of benzyl alcohol or the alkali metal salt of a C₁-C₆-alkyl alcohol into a compound of the formula I with R¹, R², X and Y having the stated meanings and, where appropriate, converting a compound of the formula I with Y not equal to hydrogen, obtained by process a) or b), in a manner known per se by acid or alkaline hydrolysis into a compound of the formula I with Y equal to hydrogen, and, where appropriate, converting a resulting compound of the formula I into a physiologically tolerated salt.

7. The use of compounds as claimed in claim 1 as reagent.

## Claims (Claims for the following Contracting State(s): ES)

1. A process for preparing a pyrimidine derivative of the formula I 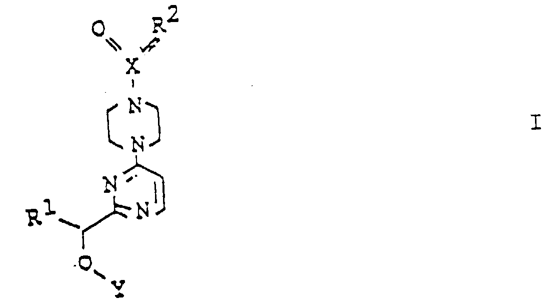 in which
R¹ is hydrogen or methyl,
R² is C₁-C₆-alkyl, C₅-C₇-cycloalkyl, unsubstituted phenyl, phenyl which is mono- or disubstituted by methyl, chlorine, fluorine, trifluoromethyl, methoxy, CH₃-SOₙ with n equal to zero, 1 or 2, or -SO₂-NH₂, where in the case of disubstitution the substituents are identical or different, 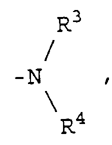 where R³ and R⁴ are identical or different and are hydrogen or C₁-C₆-alkyl, or is pyridyl or, if X is a carbon atom, is also hydrogen,
X is a carbon atom or SO, and
Y is hydrogen, and the physiologically tolerated salts thereof, which comprises
a) reacting a compound of the formula II 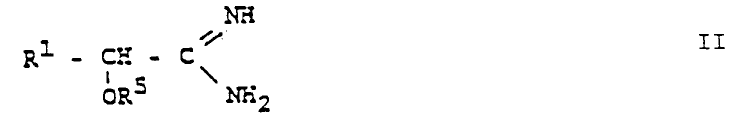 in which R¹ has the meaning stated for formula I, and R⁵ is a suitable ether protective group, or the acid addition salt thereof, with a compound of the formula III 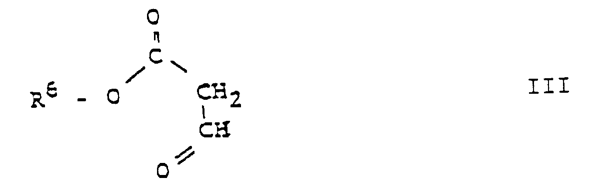 in which R⁶ is methyl or ethyl, or one of the alkali metal salts thereof, to give a compound of the formula IV 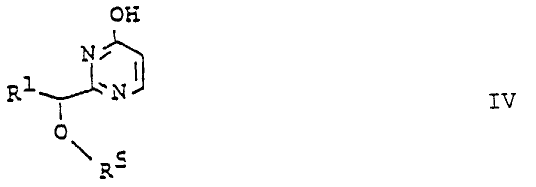 in which R¹ has the meanings stated for formula I and R⁵ has the meanings stated for formula II, converting a resulting compound of the formula IV with an inorganic acid chloride into a pyrimidine derivative of the formula V 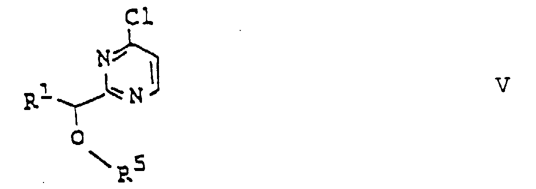 in which the radicals R¹ have the meaning stated for formula I and R⁵ has the meaning stated for formula II,
and subsequently,
α) reacting a resulting compound of the formula V with a piperazine derivative of the formula VI 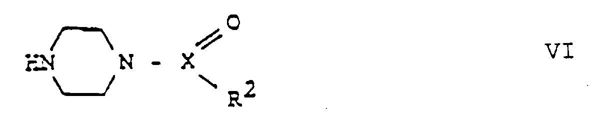 in which R² and X have the meanings stated for formula I, to give a compound of the formula VII 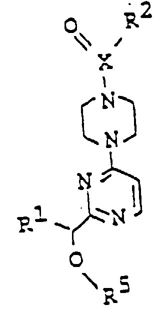 in which R¹, R² and X have the meaning stated for formula I and R⁵ has the meaning stated for formula II, or
β) converting a resulting compound of the formula V with unsubstituted piperazine into a compound of the formula VIII 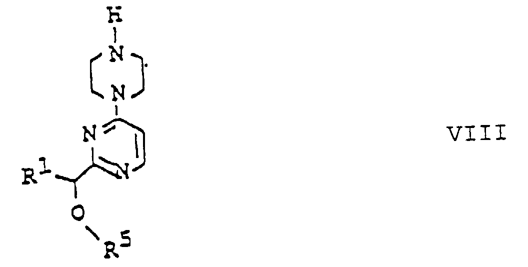 in which R¹ has the meaning stated for formula I and R⁵ has the meaning stated for formula II, and converting the resulting compound VIII with an acid chloride of the formula IX 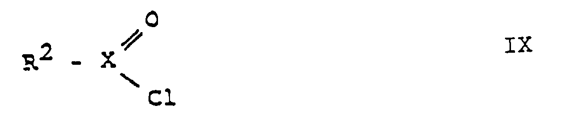 in which R² and X have the meaning stated for formula I, into a compound of the formula VII with the stated meanings of the substituents, and converting a compound of the formula VII obtained as in α) or β) where appropriate in a manner known per se by an ether-cleaving reagent, or by catalytic hydrogenation, when R⁵ has the meaning of benzyl, into a compound of the formula I,
b) converting a compound of the formula X 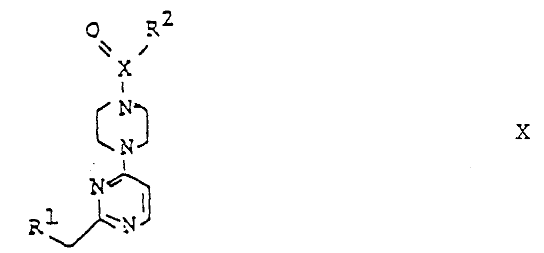 in which R¹, R² and X have the meanings stated for formula I,
with a halogenating agent into a compound of the formula XI 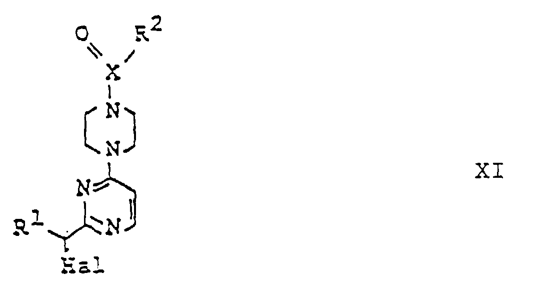 in which the substituents R¹, R² and X have the meanings stated for formula I, and Hal is Cl or Br, and converting a resulting compound of the formula XI either by direct hydrolysis or by reaction with an alkali metal salt of acetic or benzoic acid or the alkali metal salt of benzyl alcohol or the alkali metal salt of a C₁-C₆-alkyl alcohol into a compound of the formula I with R¹, R², X and Y having the stated meanings and, where appropriate, converting a compound of the formula I with Y not equal to hydrogen, obtained by process a) or b), in a manner known per se by acid or alkaline hydrolysis into a compound of the formula I with Y equal to hydrogen, and, where appropriate, converting a resulting compound of the formula I into a physiologically tolerated salt.

2. The process as claimed in claim 1, wherein a compound of the formula I in which
R¹ is hydrogen,
R² is C₁-C₃-alkyl, unsubstituted phenyl or mono- or disubstituted phenyl, where the substituents have the stated meanings, and
X and Y have the stated meanings,
or the physiologically tolerated salts thereof, is prepared.

3. The process as claimed in claim 1, wherein a compound of the formula I in which
R¹ is hydrogen,
R² is 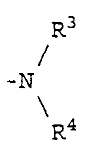 where R³ and R⁴ are identical or different and are methyl or ethyl,
X is SO, and
Y has the stated meaning,
or the physiologically tolerated salts thereof, is prepared.

4. The process as claimed in claim 1, wherein a compound of the formula I in which
R¹ is hydrogen,
R² is pyridyl,
X is a carbon atom, and
Y has the stated meaning,
or the physiologically tolerated salts thereof, is prepared.

5. The process as claimed in claim 1, wherein a compound of the formula I in which R¹ is hydrogen, R² is 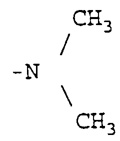 X is SO and Y is hydrogen, or the physiologically tolerated salts of this compound, is prepared.

6. The use of compounds obtainable as claimed in claim 1 as reagent.

## Revendications (Revendications pour l'(les) Etat(s) contractant(s) suivant(s): AT, BE, CH, DE, DK, FR, GB, GR, IT, LI, LU, NL, SE)

1. Dérivés de pyrimidine de formule I 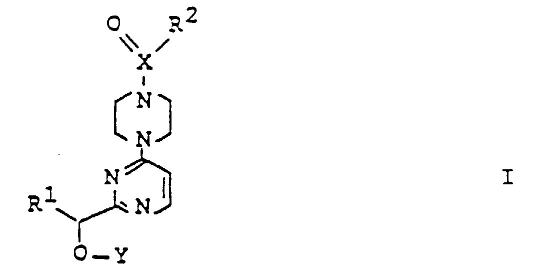 dans laquelle
R¹ représente un atome d'hydrogène ou le groupe méthyle,
R² représente un groupe alkyle en C₁-C₆, cycloalkyle en C₅-C₇, phényle non substitué, phényle mono- ou disubstitué par un ou des atomes de chlore ou de fluor ou radicaux méthyle, trifluorométhyle, méthoxy, CH₃-SOₙ, n étant égal à 0, 1 ou 2, ou -SO₂-NH₂, dans le cas de la disubstitution, les substituants étant identiques ou différents, un groupe 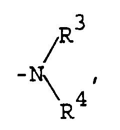 R³ et R⁴ étant identiques ou difrents et représentant un atome d'hydrogène ou un radical alkyle en C₁-C₆, ou représente le groupe pyridyle, ou bien, lorsque X est un atome de carbone, réprésente également un atome d'hydrogène,
X est un atome de carbone ou SO et
Y est un atome d'hydrogène,
ainsi que leurs sels physiologiquement acceptables.

2. Dérivés de pyrimidine selon la revendication 1, caractérisés en ce que, dans la formule I
R¹ est un atome d'hydrogène,
R² représente un groupe alkyle en C₁-C₃, phényle non substitué ou phényle mono- ou disubstitué, les substituants ayant les significations indiquées, et
X et Y ont les significations indiquées.

3. Dérivés de pyrimidine selon la revendication 1, caractérisés en ce que, dans la formule I
R¹ est un atome d'hydrogène,
R² représente un groupe 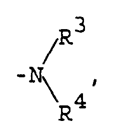 R³ et R⁴ étant identiques ou ou différents et représentant le groupe méthyle ou éthyle,
X représente SO et
Y a la signification indiquée.

4. Dérivés de pyrimidine selon la revendication 1, caractérisés en ce que, dans la formule I
R¹ est un atome d'hydrogène,
R² représente le groupe pyridyle,
X représente un atome de carbone et Y a la signification indiquée.

5. Dérivé de pyrimidine de formule I indiquée dans la revendication 1, dans laquelle
R¹ représente un atome d'hydrogène,
R² représente 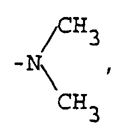
X représente SO et
Y est un atome d'hydrogène,
et sels physiologiquement acceptables de ce composé.

6. Procédé pour la préparation d'un composé selon la revendication 1, caractérisé en ce que, d'une façon connue en soi
a) on fait réagir un composé de formule II 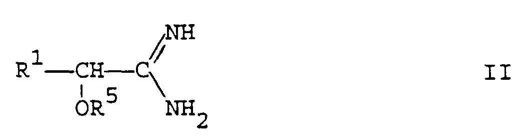 dans laquelle R¹ a la signification indiquée à propos de la formule I et R⁵ représente un groupe protecteur approprié en fonction éther, ou un sel d'addition avec un acide de ce composé, avec un composé de formule III 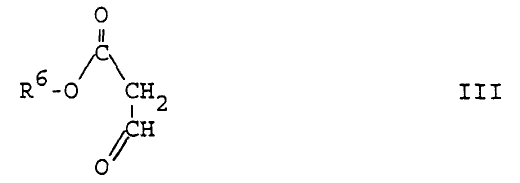 dans laquelle R⁶ représente le groupe méthyle ou éthyle, ou un de ses sels alcalins, pour aboutir à un composé de formule IV 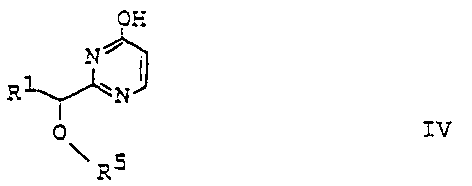 dans laquelle R¹ a la signification donnée à propos de la formule I et R⁵ a la signification donnée à propos de la formule II,
on convertit un composé de formule IV obtenu, à l'aide d'un chlorure d'acide minéral, en un dérivé de pyrimidine de formule V 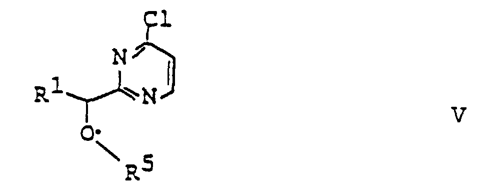 dans laquelle le radical R¹ a la signification donnée à propos de la formule I et le radical R⁵ a la signification donnée à propos de la formule II,
et ensuite
α) on fait réagir un composé de formule V obtenu, avec un dérivé de pipérazine de formule VI 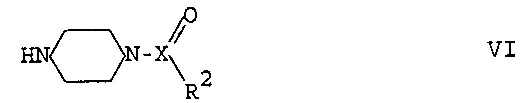 dans laquelle R² et X ont les significations données à propos de la formule I, pour aboutir à un composé de formule VII 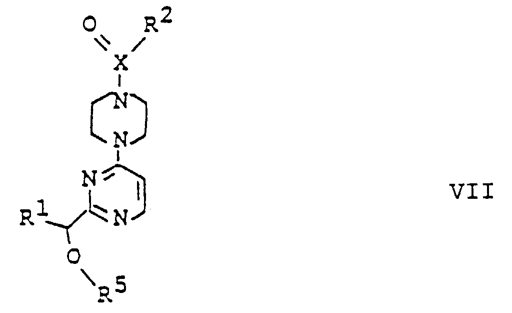 dans laquelle R¹, R² et X ont les significations données à propos de la formule I et R⁵ a la signification donnée à propos de la formule II, ou
β) on convertit un composé de formule V obtenu, à l'aide de pipérazine non substituée, en un composé de formule VIII 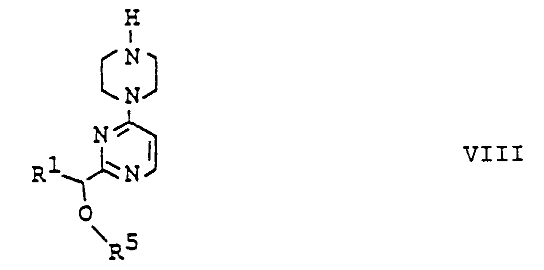 dans laquelle R¹ a la signification donnée à propos de la formule I et R⁵ a la signification donnée à propos de la formule II, et on convertit le composé VIII obtenu, à l'aide d'un chlorure d'acide de formule IX 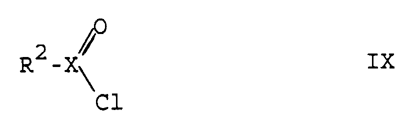 dans laquelle R² et X ont les significations données à propos de la formule I, en un composé de formule VII ayant les significations indiquées des substituants, et éventuellement on convertit un composé de formule VII, obtenu selon α) ou β), d'une façon connue en soi, au moyen d'un réactif coupant un éther, ou par hydrogénation catalytique, lorsque R⁵ a la signification du groupe benzyle, en un composé de formule I,
b) on convertit un composé de formule X 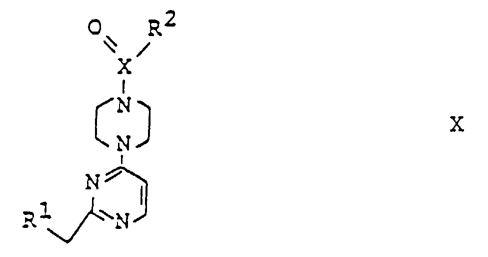 dans laquelle R¹, R² et X ont les significations données à propos de la formule I,
à l'aide d'un agent d'halogénation, d'une façon connue en soi, en un composé de formule XI 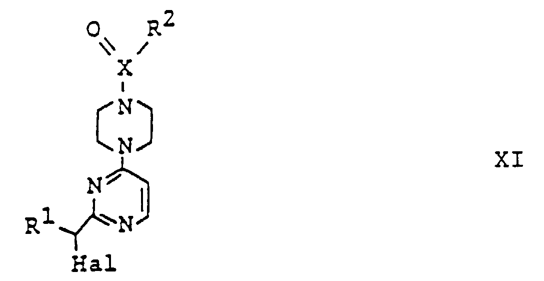 dans laquelle les substituants R¹, R² et X ont les significations données à propos de la formule I, et Hal représente Cl ou Br,
et on convertit un composé de formule XI obtenu, soit par hydrolyse directe, soit par la réaction avec un sel alcalin de l'acide acétique ou benzoïque, ou un sel alcalin de l'alcool benzylique ou un sel alcalin d'un alcool alkylique en C₁-C₆, en un composé de formule I dans lequel R¹, R², X et Y ont les significations données, et éventuellement on convertit un composé de formule I, obtenu selon le procédé a) ou b), dans lequel Y est différent d'un atome d'hydrogène, d'une façon connue en soi, par hydrolyse acide ou alcaline, en un composé de formule I dans lequel Y représente un atome d'hydrogène, et éventuellement on convertit un composé de formule I obtenu en un sel physiologiquement acceptable.

7. Utilisation des composés selon la revendication 1 en tant que réactif.

## Revendications (Revendications pour l'(les) Etat(s) contractant(s) suivant(s): ES)

1. Procédé pour la préparation de dérivés de pyrimidine de formule I 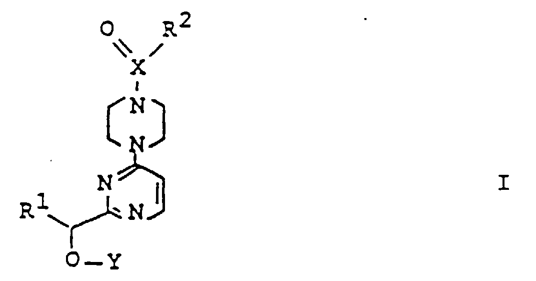 dans laquelle
R¹ représente un atome d'hydrogène ou le groupe méthyle,
R² représente un groupe alkyle en C₁-C₆, cycloalkyle en C₅-C₇, phényle non substitué, phényle mono- ou disubstitué par un ou des atomes de chlore ou de fluor ou radicaux méthyle, trifluorométhyle, méthoxy, CH₃-SOₙ, n étant égal à 0, 1 ou 2, ou -SO₂-NH₂, dans le cas de la disubstitution, les substituants étant identiques ou différents, un groupe 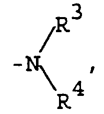 R³ et R⁴ étant identiques ou difrents et représentant un atome d'hydrogène ou un radical alkyle en C₁-C₆, ou représente le groupe pyridyle, ou bien, lorsque X est un atome de carbone, réprésente également un atome d'hydrogène,
X est un atome de carbone ou SO et
Y est un atome d'hydrogène,
ainsi que leurs sels physiologiquement acceptables, caractérisé en ce que
a) on fait réagir un composé de formule II 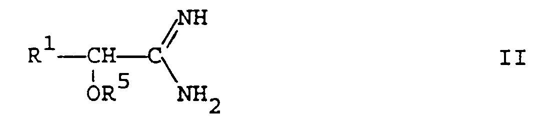 dans laquelle R¹ a la signification indiquée à propos de la formule I et R⁵ représente un groupe protecteur approprié en fonction éther, ou un sel d'addition avec un acide de ce composé, avec un composé de formule III 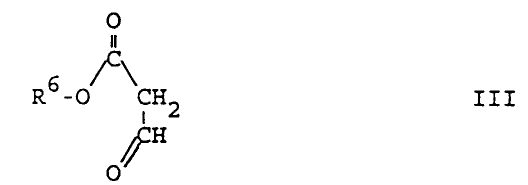 dans laquelle R⁶ représente le groupe méthyle ou éthyle, ou un de ses sels alcalins, pour aboutir à un composé de formule IV 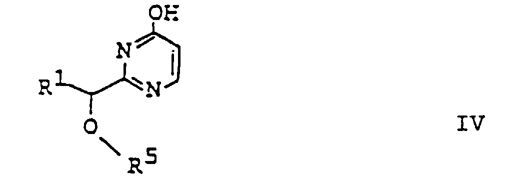 dans laquelle R¹ a la signification donnée à propos de la formule I et R⁵ a la signification donnée à propos de la formule II,
on convertit un composé de formule IV obtenu, à l'aide d'un chlorure d'acide minéral, en un dérivé de pyrimidine de formule V 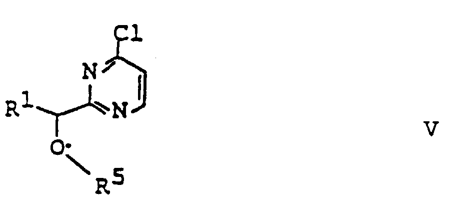 dans laquelle le radical R¹ a la signification donnée à propos de la formule I et le radical R⁵ a la signification donnée à propos de la formule II,
et ensuite
α) on fait réagir un composé de formule V obtenu, avec un dérivé de pipérazine de formule VI 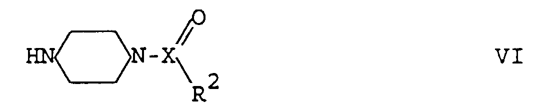 dans laquelle R² et X ont les significations données à propos de la formule I, pour aboutir à un composé de formule VII 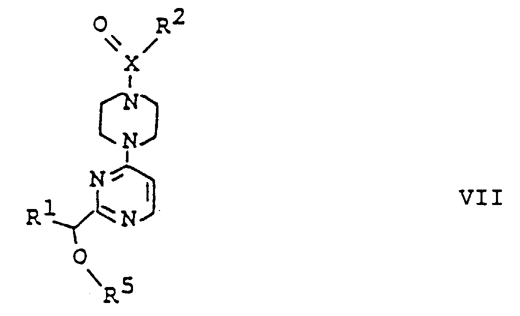 dans laquelle R¹, R² et X ont les significations données à propos de la formule I et R⁵ a la signification donnée à propos de la formule II, ou
β) on convertit un composé de formule V obtenu, à l'aide de pipérazine non substituée, en un composé de formule VIII 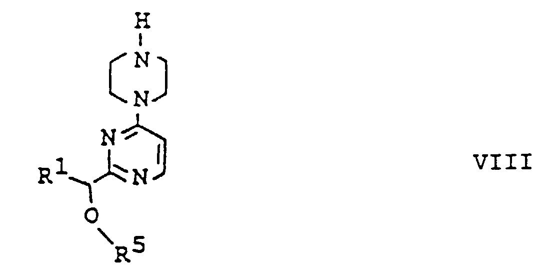 dans laquelle R¹ a la signification donnée à propos de la formule I et R⁵ a la signification donnée à propos de la formule II, et on convertit le composé VIII obtenu, à l'aide d'un chlorure d'acide de formule IX 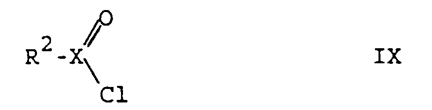 dans laquelle R² et X ont les significations données à propos de la formule I, en un composé de formule VII ayant les significations indiquées des substituants, et éventuellement on convertit un composé de formule VII, obtenu selon α) ou β), d'une façon connue en soi, au moyen d'un réactif coupant un éther, ou par hydrogénation catalytique, lorsque R⁵ a la signification du groupe benzyle, en un composé de formule I,
b) on convertit un composé de formule X 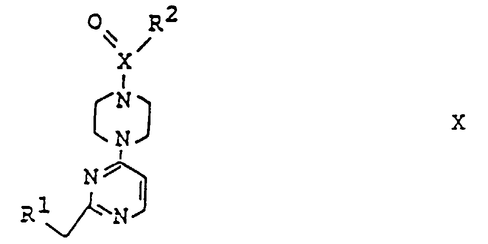 dans laquelle R¹, R² et X ont les significations données à propos de la formule I,
à l'aide d'un agent d'halogénation, d'une façon connue en soi, en un composé de formule XI 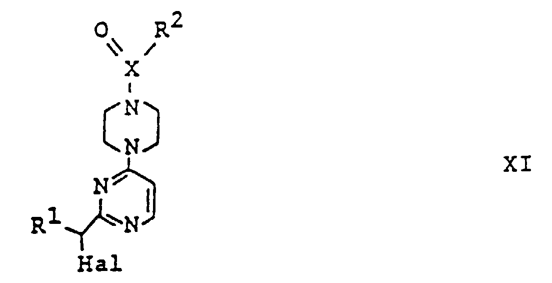 dans laquelle les substituants R¹, R² et X ont les significations données à propos de la formule I, et Hal représente Cl ou Br,
et on convertit un composé de formule XI obtenu, soit par hydrolyse directe, soit par la réaction avec un sel alcalin de l'acide acétique ou benzoique, ou un sel alcalin de l'alcool benzylique ou un sel alcalin d'un alcool alkylique en C₁-C₆, en un composé de formule I dans lequel R¹, R², X et Y ont les significations données, et éventuellement on convertit un composé de formule I, obtenu selon le procédé a) ou b), dans lequel Y est différent d'un atome d'hydrogène, d'une façon connue en soi, par hydrolyse acide ou alcaline, en un composé de formule I dans lequel Y représente un atome d'hydrogène, et éventuellement on convertit un composé de formule I obtenu en un sel physiologiquement acceptable.

2. Procédé selon la revendication 1, caractérisé en ce que l'on prépare un composé de formule I dans laquelle
R¹ est un atome d'hydrogène,
R² représente un groupe alkyle en C₁-C₃, phényle non substitué ou phényle mono- ou disubstitué, les substituants ayant les significations indiquées, et
X et Y ont les significations indiquées, ou un de ses sels physiologiquement acceptables.

3. Procédé selon la revendication 1, caractérisé en ce que l'on prépare un composé de formule I dans laquelle
R¹ est un atome d'hydrogène,
R² représente un groupe 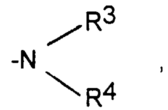 R³ et R⁴ étant identiques ou différents et représentant le groupe méthyle ou éthyle,
X représente SO et
Y a la signification indiquée, ou un de ses sels physiologiquement acceptables.

4. Procédé selon la revendication 1, caractérisé en ce que l'on prépare un composé de formule I dans laquelle
R¹ est un atome d'hydrogène,
R² représente le groupe pyridyle,
X représente un atome de carbone et
Y a la signification indiquée, ou un de ses sels physiologiquement acceptables.

5. Procédé selon la revendication 1, caractérisé en ce que l'on prépare un composé de formule I dans laquelle
R¹ représente un atome d'hydrogène,
R² représente 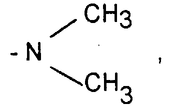
X représente SO et
Y est un atome d'hydrogène, ou un de ses sels physiologiquement acceptables.

6. Utilisation d'un composé préparé selon la revendication 1 en tant que réactif.
